(19) Europäisches Patentamt
European Patent Office
Office européen des brevets

(11) Numéro de publication : **0 482 966 A2**

# (12) DEMANDE DE BREVET EUROPEEN

(21) Numéro de dépôt : **91402471.6**

(22) Date de dépôt : **18.09.91**

(51) Int. Cl.[5] : **C07K 7/06,** C07K 7/08, C07K 7/02, C07K 7/50, C07K 7/64, A61K 37/64

(30) Priorité : **26.09.90 FR 9011842**

(43) Date de publication de la demande : **29.04.92 Bulletin 92/18**

(84) Etats contractants désignés : **AT BE CH DE DK ES FR GB GR IT LI LU NL SE**

(71) Demandeur : **ADIR ET COMPAGNIE**
**1 rue Carle Hébert**
**F-92415 Courbevoie Cédex (FR)**

(72) Inventeur : **Fauchere, Jean-Luc**
**18 rue Beffroy**
**F-92200 Neuilly (FR)**
Inventeur : **Thurieau, Christophe**
**120 rue Silly**
**F-92100 Boulogne (FR)**
Inventeur : **Picard, Isabelle**
**18 rue Eugène Gibez**
**F-75015 Paris (FR)**
Inventeur : **Verbeuren, Tony**
**60 bis rue Aristide Briand**
**F-78540 Vernouillet (FR)**

(54) **Dérivés stabilisés de fragments d'hirudine, leur procédé de préparation et leurs compositions pharmaceutiques.**

(57) L'invention concerne les dérivés de formule (I) :

$$X\text{-}A_1\text{-}A_2\text{-}A_3\text{-}A_4\text{-}A_5\text{-}A_5\text{-}A_7\text{-}A_5\text{-}A_9\text{-}A_{10}\text{-}Y \qquad (I)$$

dans laquelle X, $A_1$, $A_2$, $A_3$, $A_4$, $A_5$, $A_5$, $A_7$, $A_5$, $A_5$, $A_{10}$ et Y sont tels que définis dans la description.
Médicaments.

EP 0 482 966 A2

La présente invention concerne de nouveaux dérivés de peptides et de pseudopeptides therapeutiquement actifs dans la cascade de coagulation sanguine, leur procédé de préparation et les compositions pharmaceutiques qui les contiennent.

Il est désormais largement connu que lorsque l'équilibre entre facteurs procoagulants et anticoagulants dans le sang est perturbé, il peut en résulter la formation d'un thrombus ou caillot de sang. Le développement d'une thrombose est favorisé essentiellement par trois facteurs pathogéniques principaux qui sont la stase ou diminution du flux sanguin, les états d'hypercoagulabilité et les lésions de l'endothélium de la paroi vasculaire.

Les anticoagulants sont des agents thérapeutiques utilisables dans le traitement des thromboses veineuses aigües, de l'embolie pulmonaire, de l'embolie artérielle des extrêmités, des thromboses artérielles comme l'infarctus du myocarde et de toutes les autres manifestations thromboemboliques.

Parmi les agents anticoagulants connus, l'hirudine, qui est un polypeptide comportant 65 acides aminés, est un inhibiteur spécifique de la thrombine isolé des glandes salivaires de la sangsue médicinale (Biochemistry 25, 4622-28, 1986).

Des variantes de l'hirudine utilisables en tant qu'inhibiteur de thrombine ont déjà été décrits. C'est le cas, par exemple, des composés décrits dans les brevets EP 209061 ou EP 332523. D'autre part, des analogues synthétiques de fragments d'hirudine à propriétés anticoagulantes ont également été décrits ; c'est le cas, par exemple, des composés revendiqués dans les brevets EP 276014, EP 291981, EP 291982 et EP 333356.

La présente invention a pour objet des peptides synthétiques nouveaux qui se sont montrés particulièrement intéressants en raison de l'intensité de leurs propriétés anticoagulantes, antithrombotiques et en tant qu'antiagrégants plaquettaires sans ce que ces effets soient accompagnés d'une action hémorragique marquée. Ces propriétés s'exercent jusqu'à des doses 10 fois plus faibles que celles des autres composés décrits dans l'art antérieur.

D'autre part, les dérivés de l'invention présentent une stabilité élevée par rapport au peptide naturel (hirudine 54-65) dans des tests de protéolyse in vitro qui sont des modèles de leur durée d'action in vivo. La puissance, la sélectivité et la stabilité de ces peptides synthétiques sont dues notamment à l'introduction dans la séquence peptidique d'acides aminés synthétiques, de liaisons pseudopetidiques, de cyclisations non cystéiniques ou d'une combinaison de ces facteurs.

L'invention concerne plus particulièrement de nouveaux dérivés peptidiques répondant à la formule générale (I) :

$$X\text{-}A_1\text{-}A_2\text{-}A_3\text{-}A_4\text{-}A_5\text{-}A_6\text{-}A_7\text{-}A_6\text{-}A_9\text{-}A_{10}\text{-}Y \qquad (I)$$

dans laquelle :

X représente -

un groupement amino terminal éventuellement substitué par 1 ou 2 groupements alkyls ($C_1$-$C_6$) linéaires ou ramifiés, un groupement acyl ($C_1$-$C_6$) linéaire ou ramifié, un groupement benzyloxycarbonyl (Z), un groupement tert-butoxycarbonyl (Boc), un groupement 9-fluorénylméthyloxycarbonyl (Fmoc), un groupement guanidinoacyl ($C_1$-$C_6$) linéaire ou ramifié tel que les groupements

$X_0$ = 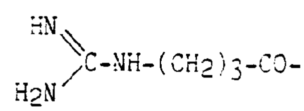 ,

ou $X'_0$ = 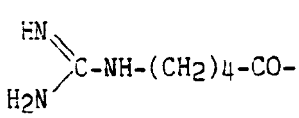 ,

un groupement guanidinoalkyl ($C_1$-$C_6$) linéaire ou ramifié, un groupement pyroglutamyl (Glp), un groupement succinyle (Suc), ou l'un quelconque des groupemenis suivants :

$X_1$ = 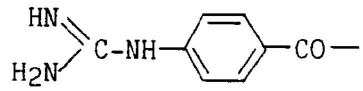

$X_2$ = 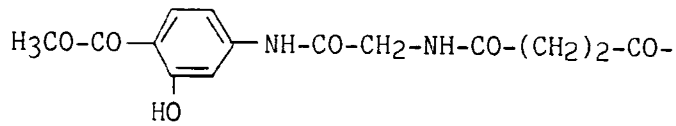

$X_3$ = 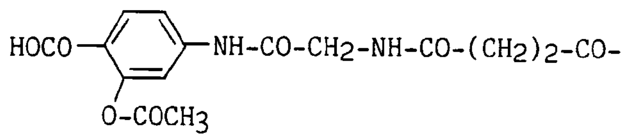

$X_4$ = 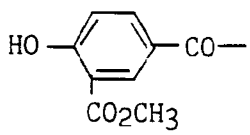

$X_5$ = 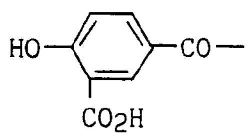

$X_6$ = 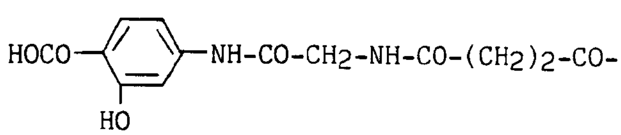

$X_7$ = 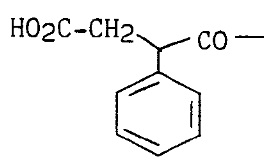

$X_8$ = 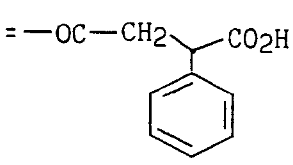

$A_1$ représente une liaison ou un résidu peptidique contenant de 1 à 3 amino-acides quelconques,

$A_2$ représente une liaison ou un résidu phénylalanine (Phe), tyrosine (Tyr), isoleucine (Ile), norvaline (Nva), (1,2,3,4)-tétrahydroisoquinoline-3-carbonyl (Tic), un résidu acide α-aminobutyrique (Abu), acide glutamique (Glu), ou un résidu d'acide aminé contenant un groupement aryle,

$A_3$ représente une liaison ou un résidu acide glutamique (Glu), acide aspartique (Asp), β-alanine (βAla) ou tyrosine (Tyr),

$A_4$ représente une liaison ou un résidu acide glutamique (Glu), acide aspartique (Asp), proline (Pro), acide 2,3-diaminopropionique (Dpr), acide 2,4-diaminobutyrique (Dab), ornithine (Orn), lysine (Lys), 2-azabicyclo [2.2.2] octane-3-carbonyl (Abo), 2-azabicyclo [2.2.1] heptane-3-carbonyl (Abh),

$A_5$ représente une liaison ou un résidu isoleucine (Ile), norvaline (Nva), phénylalanine (Phe), proline (Pro), ornithine (Orn) ou acide 2,3-diaminopropionique (Dpr),

$A_5$ représente un résidu proline (Pro), isoleucine (Ile), norvaline (Nva), phénylalanine (Phe), ornithine (Orn), 2-azabicyclo [2.2.2] octane-3-carbonyl (Abo), 2-azabicyclo [2.2.1] heptane-3-carbonyl (Abh), octahydroindole-2-carbonyl (Oic) ou 3,4-déhydroproline (dhPro),

$A_7$ représente un résidu acide glutamique (Glu) ou acide aspartique (Asp),

$A_5$ représente un résidu acide glutamique (Glu), acide aspartique (Asp), acide 2,3-diaminopropionique (Dpr), acide 2,4-diaminobutyrique (Dab) ou β-alanine (βAla),

$A_9$ représente un résidu tyrosine (Tyr) éventuellement protégé par un groupement benzyle (Tyr(Bzl)) ou acétyle (Tyr($COCH_3$)), phénylalanine (Phe), alanine (Ala), isoleucine (Ile), norvaline (Nva), p-carboxyphénylalanine (Pcp), 3-carboxytyrosine ou 4-0-acétyl-3-carboxytyrosine, (p-$CH_2PO_3H$) phénylalanine ((p$CH_2PO_3H$)Phe),

$A_{10}$ représente une liaison, un résidu leucine (Leu), valine (Val), β-naphtylalanine (Nal), cyclohexylalanine (Cha), β-(2-thiényl)alanine (Thi), octahydroindole-2-carbonyl (Oic), un résidu dipeptidique tel que Leu-Glu, Leu-Pro, Leu-βAla, Val-Glu, Nal-Glu, Cha-Glu- Thi-Glu, Oic-Glu, leucine-acide 4-aminobutyrique (Leu-4Abu), ou un résidu tripeptidique tel que Nal-Nal-Leu,

Y représente

– un groupement carboxy terminal éventuellement substitué par un groupement alkoxy ($C_1$-$C_6$) linéaire ou ramifié ou un groupement amino lui-même éventuellement substitué par un ou deux groupements alkyles ($C_1$-$C_6$) linéaires ou ramifiés,

ou bien

– un groupement carboxy terminal réduit en alcool correspondant (ol),

<u>et comprenant au moins</u> :

– un résidu d'amino-acide non naturel tel que le 2-azabicyclo [2.2.2] octane-3-carbonyl (Abo), le 2-azabicyclo [2.2.1] heptane-3-carbonyl (Abh), l'octahydroindole-2-carbonyl (Oic),

et/ou

– une liaison pseudopeptidique telle que -$CH_2$-NH-, -$CH_2$-S-, -$CH_2$-SO-, -$CH_2$-$SO_2$-, -NH-CO-, -CH=CH- remplaçant une liaison peptidique -CO-NH-,

et/ou

– une cyclisation non cystéinique entre les chaînes latérales de deux amino-acides, ou entre un groupement amino terminal et une chaîne latérale d'un amino-acide ou entre un groupement carboxy terminal et une chaîne latérale d'un amino-acide ou entre un groupement amino terminal et un groupement carboxy terminal,

leurs sels d'addition à un acide ou à une base pharmaceutiquement acceptable,
chaque amino-acide de la séquence peptidique étant optiquement pur et le carbone a de chaque amino-acide avant la configuration D ou L.

Parmi les acides pharmaceutiquement acceptables on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables on peut citer à titre non limitatif l'hydroxyde de sodium, l'hydroxyde de potassium, la triéthylamine, la tertbutylamine, etc...

L'invention s'étend aussi au procédé de préparation des dérivés de formule (I) qui peuvent être obtenus par différentes méthodes telles que la synthèse séquentielle sur phase solide, la synthèse de fragments et leur couplage en solution, la synthèse enzymatique, la synthèse génétique par clonage et expression de gènes dans des bactéries transformées ou par des combinaisons diverses de ces techniques.

Les méthodes générales de la synthèse des peptides sur phase solide ont été décrites par B.W. ERICKSON et R.B. MERRIFIELD ("The Proteins", Solid-Phase Peptide Synthesis, 3ème édition, 257-527, 1976).

La synthèse sur phase solide peut se réaliser sur un automate qui effectue de façon répétitive et programmable des cycles de déprotection, couplage et lavages nécessaires à l'introduction séquentielle des acides aminés dans la chaîne peptidique. L'acide aminé, de préférence C-terminal est fixé sur une résine conventionnellement utilisée pour la préparation de polypeptides, de préférence un polystyrène réticulé à l'aide de 0,5 à 3,0 % de divinylbenzène et muni de restes activés tels que le chlorométhylène ou l'hydroxyméthylène qui permettent la fixation covalente du premier acide aminé sur la résine. Le choix approprié de la résine permet la fixation d'une fonction C-terminale acide carboxylique, amide ou alcool.

Les acides aminés sont alors introduits un à un dans l'ordre déterminé par l'opérateur. Chaque cycle de synthèse correspondant à l'introduction d'un acide aminé, comporte une déprotection, de préférence N-terminale de la chaîne peptidique, des lavages successifs destinés à éliminer les réactifs ou à gonfler la résine, un couplage avec activation de l'acide aminé et de nouveaux lavages. Chacune de ces opérations est suivie d'une filtration réalisée grâce à la présence d'un verre fritté incorporé au réacteur dans lequel s'effectue la synthèse.

Les réactifs de couplage utilisés sont des réactifs classiques de la synthèse peptidique comme le dicyclohexylcarbodiimide (DCC) et l'hydroxybenzotriazole (HOBT) ou le benzotriazol-1-yl-oxytris (diméthylamino) phosphonium hexafluorophosphate (BOP) ou encore le diphenylphosphorylazide (DPPA).

Des activations par formation d'anhydrides mixtes sont également possibles.

Chaque acide aminé est introduit dans le réacteur en quadruple excès environ, par rapport au degré de substitution de la résine et en quantité environ équivalente par rapport aux agents de couplage. La réaction de couplage peut être vérifiée à chaque étape de la synthèse par le test de réaction à la ninhydrine décrit par E. KAISER et Coll. (Analyt. Biochem., 34, 595, 1970).

Après assemblage de la chaîne peptidique sur la résine, un traitement par un acide fort tel que l'acide trifluoroacétique, ou l'acide fluorhydrique en présence d'anisole, d'éthanedithiol ou de 2-méthylindole sert à séparer le peptide de la résine ainsi qu'à libérer éventuellement le peptide de ses groupes protecteurs. Le composé est alors purifié par des techniques classiques de purification, notamment chromatographiques.

La cyclisation totale ou partielle des composés de l'invention est réalisée en solution de préférence à travers deux fonctions déprotégées de façon sélective et capables de former une liaison covalente comme une liaison amide.

Les peptides de la présente invention peuvent être également obtenus par couplage en solution de fragments peptidiques sélectivement protégés, qui peuvent être préparés soit sur phase solide, soit en solution. L'emploi des groupes protecteurs et la mise à profit de leur stabilité différentielle est analogue aux méthodes sur phase solide à l'exception de l'attachement de la chaîne peptidique sur la résine. Le groupe carboxylique C-terminal est protégé, par exemple, par un ester méthylique ou une fonction amide. Les méthodes d'activation lors des couplages sont également analogues à celles employées dans la synthèse sur phase solide.

La synthèse de peptides contenant des liaisons pseudopeptidiques telles que $-CH_2-NH-$, $-CH_2-S-$, $-CH_2-SO-$, $-CH_2-SO_2-$, -NH-CO-, -CH=CH- est effectuée soit par les méthodes en solution soit de façon combinée avec la synthèse sur phase solide en utilisant les méthodes classiques de la chimie organique. Ainsi, par exemple, l'introduction de la liaison $-CH_2-NH$ se fait en préparant en solution l'aldéhyde Fmoc-NH-CHR-CHO selon la technique décrite par FEHRENTZ et CASTRO (Synthesis, 676-678, 1983) et en le condensant avec la chaîne peptidique croissante soit sur phase solide selon la technique décrite par SASAKI et COY (Peptides, 8, 119-121, 1988), soit en solution.

Les composés de formule (I) possèdent des propriétés pharmacologiques très intéressantes.

Ils sont doués de propriétés anticoagulantes et antithrombotiques et peuvent ainsi être utilisés pour prévenir les complications postthromboemboliques par dissolution des caillots ou en tant qu'agents préventifs de l'extension du processus thrombotique en les utilisant comme anticoagulants d'action directe et rapide. Leurs propriétés d'inhibition de la voie d'activation plaquettaire médiée par la thrombine permettent d'envisager leur utilisation en tant qu'inhibiteur des étapes de l'interaction des plaquettes sanguines avec la paroi vasculaire impliquées dans les phénomènes de thrombose et d'athérosclérose.

La présente invention a également pour objet les compositions pharmaceutiques renfermant comme principe actif au moins un composé de formule générale (I) ou un de ses sels d'addition à un acide ou à une base pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, crèmes, pommades, gels dermiques, les aérosols.

La posologie varie selon l'âge et le poids du patient, la nature et la sévérité de l'affection ainsi que la voie d'administration.

Celle-ci peut être orale, nasale, rectable ou parentérale. D'une manière générale, elle s'échelonne entre 0,2 et 100 mg pour un traitement en une ou plusieurs prises par 24 heures.

Les exemples suivants illustrent l'invention et ne la limitent en aucune façon.

Les acides aminés dont les abréviations commencent par une lettre majuscule sont de configuration L.

Les acides aminés dont les abréviations commencent par une lettre minuscule sont de configuration D.

L'acide aminé nommé Abo est de configuration 3S.

La lettre $\Psi$ indique la présence d'une liaison pseudopeptidique dont la nature est indiquée entre parenthèses.

**EXEMPLE 1 : Z-Asp-Phe-Glu-Abo-Ile-Pro-Glu-Asp-Tyr-Leu-OH**

Le composé de l'exemple 1 est synthétisé à partir de 2 g d'une résine substituée par 0,33 mmole/g de Fmoc-Leu-OH et suivant le protocole répétitif suivant :

| N° opération | Fonction | Solvant/Réactif | Répétition/temps |
|---|---|---|---|
| 1 | lavage | DMF | 2 × 2 min |
| 2 | déprotection | 20 % pipéridine/DMF | 1 × 5 min |
| 3 | déprotection | 20 % pipéridine/DMF | 1 × 15 min |
| 4 | lavage | DMF | 3 × 2 min |
| 5 | lavage | dichlorométhane | 3 × 2 min |
| 6 | couplage | acide aminé protégé activé | 1 × 90 min |
| 7 | lavage | DMF | 3 × 2 min |
| 8 | lavage | isopropylique alcool | 3 × 2 min |
| 9 | lavage | dichlorométhane | 3 × 2 min |

Chacune de ces opérations effectuée dans 30 ml de solvant, sous agitation et à témpérarture ambiante, est suivie d'une filtration à travers un verre fritté incorporé à la cellule de verre (réacteur) dans laquelle la synthèse progresse. Le filtre retient la résine sur laquelle est fixée la chaîne peptidique croissante.

Les acides aminés protégés choisis ont été introduits dans l'ordre suivant : Fmoc-Tyr(tBu)-OH, Fmoc-Asp-(OtBu)-OH, Fmoc-Glu(OtBu)-OH, Fmoc-Pro-OH, Fmoc-Ile-OH, Fmoc-Abo-OH, Fmoc-Glu(OtBu)-OH, Fmoc-Phe-OH, Z-Asp(OtBu)-OH.

Le 2-aza-3-carboxy-bicyclo[2.2.2]-octane (Abo) est un acide aminé non codé décrit dans le brevet EP 51020. Le dérivé Fmoc-Abo-OH est obtenu par traitement de l'Abo par le 9-fluorénylméthyl chloroformate (Fmoc-Cl) dans un mélange $Na_2CO_3$(10 %)/dioxane (2/1) pendant 4 heures à température ambiante, puis extraction, après acidification de la phase aqueuse par de l'acétate d'éthyle et recristallisation dans un mélange éther/pentane.

L'activation en vue du couplage (opération 6) est obtenue à chaque cycle par dissolution de 4 équivalents (2.64 mmoles) de l'acide aminé protégé avec 360 mg d'HOBt dans 30 ml de DMF, puis après 30 minutes à température ambiante, par addition de 618 mg de DCC. Cette solution est alors immédiatement introduite dans la cellule de réaction avec 10 ml de dichlorométhane.

A la fin des neuf cycles correspondant à la fixation séquentielle de neuf acides aminés, on a ainsi obtenu avec la leucine C-terminale, un décapeptide protégé sur ses chaînes latérales et fixé sur la résine. Celle-ci est alors traitée par un mélange d'acide trifluoroacétique (18 ml), dichlorométhane (1 ml) et anisol (1 ml) pendant 90 minutes à température ambiante. Le filtrat et les solvants de lavage de la résine (3 X 20 ml dichlorométhane) sont réunis et évaporés à sec. Le produit est suspendu dans l'éther, filtré et séché puis purifié par CLHP préparative, sur colonne $C_{18}$ (diamètre interne : 47 mm, longueur : 300 mm) et lyophilisé.

L'analyse du produit obtenu est réalisé après décomposition de celui-ci en acides aminés par hydrolyse dans de l'acide chlorhydrique 6N pendant 18 heures à 110°C et dosage quantitatif des acides aminés obtenus par CLHP.

| | Asp | Ile | Glu | Leu + Abo | Phe | Pro | Tyr |
|---|---|---|---|---|---|---|---|
| calculé | 2 | 1 | 2 | 2 | 1 | 1 | 1 |
| trouvé | 1,98 | 0,92 | 2,15 | 1,80 | 0,95 | 1,11 | 0,90 |

Spectre de masse (FAB) : $MH^+$, $m/_z$ = 1411

<u>EXEMPLE 2</u> : Fmoc-Asp-Phe-Glu-Dpr-Ile-Abo-Glu-Glu-Tyr Ψ($CH_2$-NH)Leu-$NH_2$

Le premier cycle est identique à celui décrit dans l'exemple 1 et permet d'obtenir l'introduction de Fmoc-Leu-OH sur une résine substituée de façon à libérer une fonction amide C-terminale lors du clivage final. Il est suivi par un demi-cycle jusqu'à l'opération 6 afin de libérer la fonction amine de la leucine.

On fait alors réagir séparément 2,3 mmoles de Fmoc-Tyr(Bzl)-OH avec 2,3 mmoles d'hexafluorophosphate de phosphonium (BOP) en présence de O,N-diméthylhydroxylamine et de triéthylamine. Le produit obtenu est isolé après lavages usuels dans le dichlorométhane et évaporation du solvant. Le produit huileux est alors repris dans de l'éther et traité par 2,5 mmoles de $LiAlH_4$ pendant 20 minutes à température ambiante. Le mélange est alors hydrolysé dans une solution de $NaHCO_3$ et l'aldéhyde formé extrait dans l'éther, lavé et le solvant est évaporé. Le produit huileux obtenu est alors introduit dans le réacteur contenant la résine en suspension dans le DMF en présence d'acide acétique. L'imine obtenue est alors réduite par addition progressive de cyanoborohydrure de sodium et l'agitation est maintenue durant 3 heures.

La synthèse se poursuit alors en utilisant le même protocole de synthèse que celui décrit dans l'exemple 1 en commençant par l'opération 7. Les amino-acides protégés sont alors introduits dans l'ordre suivant : Fmoc-Glu(OBzl)-OH, Fmoc-Glu(OtBu)-OH, Fmoc-Abo-OH, Fmoc-Ile-OH, Fmoc-Dpr(Boc)-OH, Fmoc-Glu(OBzl)-OH, Fmoc-Phe-OH, Fmoc-Asp(OBzl)-OH.

Après l'opération 9 du dernier cycle, le produit est séparé de la résine et purifié en utilisant les mêmes techniques que dans l'exemple 1. Le produit obtenu est alors cyclisé après dissolution dans le DMF et traitement par 4 équivalents de BOP et 12 équivalents d'éthyle diisopropyle amine pendant 2 heures à température ambiante.

Après évaporation du solvant, le produit cyclisé est purifié par chromatographie, puis déprotégé de ses groupes protecteurs à l'exception de Fmoc par hydrogénation catalytique (Pd/C).

Après filtration du catalyseur, le produit attendu est purifié par chromatographie liquide préparative. Sa pureté est contrôlée de la même façon que dans l'exemple 1.

<u>Analyse</u> :

| | Asp | Phe | Glu | Dpr | Ile | Abo |
|---|---|---|---|---|---|---|
| calculé | 1 | 1 | 3 | 1 | 1 | 1 |
| trouvé | 0,97 | 0,98 | 3,03 | 0,99 | 0,95 | 0,99 |

<u>Spectre de masse</u> (FAB) : $MH^+$, $m/_z$ = 1469

Les exemples suivants ont été préparés en utilisant les modes opératoires décrits dans les exemples 1 ou 2.

**<u>EXEMPLE 3</u> : Z-Asp-Phe-Glu-Abo-Ile-Abo-Glu-Asp-Tyr-Leu-OH**

<u>Analyse</u> :

| | Asp | Phe | Glu | Ile + Abo | Tyr | Leu |
|---|---|---|---|---|---|---|
| calculé | 2 | 1 | 2 | 3 | 1 | 1 |
| trouvé | 2,10 | 0,96 | 2,19 | 2,85 | 1,10 | 1,09 |

<u>Spectre de masse</u> (FAB) : $MH^+$, $m/_z$ = 1450

**<u>EXEMPLE 4</u> : nal-Asp-Phe-Glu-Abo-Ile-Pro-Glu-Asp-Tyr-Leu, trifluoroacétate**

<u>Analyse</u> :

| | nal | Asp | Phe | Glu | Ile | Pro | Tyr | Leu + Abo |
|---|---|---|---|---|---|---|---|---|
| calculé | 1 | 2 | 1 | 2 | 1 | 1 | 1 | 2 |
| trouvé | 1,07 | 1,97 | 1,01 | 2,14 | 0,96 | 1,07 | 0,90 | 1,90 |

Spectre de masse (FAB) : $MH^+$, $m/_z$ = 1474

**EXEMPLE 5 : Z-Asp-Phe-Glu-Abo-Ile-Pro-Glu-Asp-Tyr Ψ($CH_2NH$) Leu-OH, trifluoroacétate**

Analyse :

| | Asp | Phe | Glu | Abo | Ile | Pro |
|---|---|---|---|---|---|---|
| calculé | 2 | 1 | 2 | 1 | 1 | 1 |
| trouvé | 2,21 | 0,95 | 2,13 | 0,90 | 0,86 | 1,18 |

Spectre de masse (FAB) : $MH^+$, $m/_z$ = 1397

**EXEMPLE 6 : Z-Asp-Tyr Ψ ($CH_2NH$) Glu-Abo-Ile-Pro-Glu-Asp-Tyr Ψ ($CH_2NH$) Leu-OH, ditrifluoroacétate**

Analyse :

| | Asp | Abo | Ile | Pro | Glu |
|---|---|---|---|---|---|
| calculé | 2 | 1 | 1 | 1 | 1 |
| trouvé | 2,16 | 0,93 | 1,00 | 0,91 | 1,08 |

Spectre de masse (FAB) : $MH^+$, $m/_z$ = 1399

**EXEMPLE 7 : Z-Asp-Phe-Glu-Abo-Ile-Pro-Glu-Asp-Tyr-nal-nal-Leu-OH**

Analyse :

| | Asp | Phe | Glu | Abo + Ile | Pro | Tyr | nal | Leu |
|---|---|---|---|---|---|---|---|---|
| calculé | 2 | 1 | 2 | 2 | 1 | 1 | 2 | 1 |
| trouvé | 2,06 | 1,02 | 2,20 | 1,82 | 1,04 | 0,91 | 1,85 | 1,1 |

Spectre de masse (FAB) : $MH^+$, $m/_z$ = 1804

**EXEMPLE 8 : H-Glu-Orn-Pro-Glu-Glu-Tyr-Leu**

Analyse :

| | Glu | Pro | Leu | Orn | Tyr |
|---|---|---|---|---|---|
| calculé | 3 | 1 | 1 | 1 | 1 |
| trouvé | 2,85 | 0,97 | 0,98 | 1,1 | 1,08 |

Spectre de masse (FAB) : $MH^+$, m/z = 875

**EXEMPLE 9 : Suc-Glu-Orn-Pro-Glu-Glu-Tyr-Leu**

Analyse :

| | Glu | Pro | Leu | Orn | Tyr |
|---|---|---|---|---|---|
| calculé | 3 | 1 | 1 | 1 | 1 |
| trouvé | 2,95 | 1,09 | 0,99 | 0,91 | 1,06 |

Spectre de masse (FAB) : $MH^+$, m/z = 975

**EXEMPLE 10 : H-Orn-Pro-Glu-Glu-Tyr-Leu**

Analyse :

| | Glu | Pro | Leu | Orn | Tyr |
|---|---|---|---|---|---|
| calculé | 2 | 1 | 1 | 1 | 1 |
| trouvé | 1,84 | 1,04 | 0,99 | 1,08 | 1,04 |

Spectre de masse (FAB) : $MH^+$, m/z = 746

**EXEMPLE 11 : Orn-Pro-Glu-Glu-Tyr**

Analyse :

| | Glu | Pro | Orn | Tyr |
|---|---|---|---|---|
| calculé | 2 | 1 | 1 | 1 |
| trouvé | 2,05 | 0,91 | 0,99 | 1,06 |

Spectre de masse (FAB) : $MH^+$, m/z = 633

**EXEMPLE 12 : H-Glu-Orn-Pro-Glu-Glu-Tyr**

Analyse :

| | Glu | Pro | Orn | Tyr |
|---|---|---|---|---|
| calculé | 3 | 1 | 1 | 1 |
| trouvé | 2,92 | 1,06 | 1,01 | 0,99 |

Spectre de masse (FAB) : $MH^+$, m/z = 761

**EXEMPLE 13** : H-Orn-Pro-Glu-Glu-Tyr

Analyse :

| | Glu | Pro | Orn | Tyr |
|---|---|---|---|---|
| calculé | 2 | 1 | 1 | 1 |
| trouvé | 1,94 | 1,00 | 1,06 | 1,00 |

Spectre de masse (FAB) : $MH^+$, m/z = 633

**EXEMPLE 14** : Suc-Dpr-Pro-Glu-Glu-Pcp-Leu-OH

**EXEMPLE 15** : Suc-Orn-Pro-Glu-Glu-Pcp-Leu-OH

**EXEMPLE 16** : Orn-Pro-Glu-Glu-Tyr-Leu

Analyse :

| | Glu | Pro | Leu | Orn | Tyr |
|---|---|---|---|---|---|
| calculé | 2 | 1 | 1 | 1 | 1 |
| trouvé | 1,92 | 1,02 | 1,00 | 1,09 | 0,97 |

Spectre de masse (FAB) : $MH^+$, m/z = 746

**EXEMPLE 17** : H-Asp-Phe-Glu-Glu-Orn-Pro-Glu-Glu-Tyr-Leu

Analyse :

| | Asp | Glu | Pro | Phe | Leu | Orn | Tyr |
|---|---|---|---|---|---|---|---|
| calculé | 1 | 4 | 1 | 1 | 1 | 1 | 1 |
| trouvé | 1,03 | 3,98 | 1,01 | 0,97 | 1,06 | 1,00 | 1,01 |

Spectre de masse (FAB) : $MH^+$, m/z = 1266

**EXEMPLE 18** : Glp-Glu-Orn-Pro-Glu-Glu-Tyr-Leu

Analyse :

| | Glu | Pro | Leu | Orn | Tyr |
|---|---|---|---|---|---|
| calculé | 4 | 1 | 1 | 1 | 1 |
| trouvé | 4,07 | 1,02 | 0,96 | 0,94 | 1,00 |

Spectre de masse (FAB) : $MH^+$, m/z = 986

**EXEMPLE 19** : $X_2$-Glu-Glu-Orn-Pro-Glu-Glu-Tyr-Leu

Analyse :

| | Glu | Gly | Pro | Leu | Orn | Tyr |
|---|---|---|---|---|---|---|
| calculé | 4 | 1 | 1 | 1 | 1 | 1 |
| trouvé | 4,07 | 0,99 | 1,03 | 0,92 | 0,96 | 1,03 |

Spectre de masse (FAB) : $MH^+$, m/z = 1310

**EXEMPLE 20 : $X_3$-Asn-Gly-Asp-Phe-Glu-Glu-Ile-Pro-Glu-Glu-Tyr(CO-$CH_3$)-Leu-OH**

**EXEMPLE 21 : $X_0$-Gly-Asp-Phe-Glu-Abo-Ile-Pro-Glu-Glu-Tyr-Leu-OH**

Analyse :

| | Asp | Glu | Gly | Phe | Leu + Ile + Abo | Tyr |
|---|---|---|---|---|---|---|
| calculé | 1 | 3 | 1 | 1 | 3 | 1 |
| trouvé | 1,00 | 3,06 | 0,95 | 1,08 | 2,88 | 1,02 |

Spectre de masse (FAB) : $M + H^+$, m/z = 1475

**EXEMPLE 22 : H-(4-Abu)-Gly-Asp-Phe-Glu-Abo-Ile-Pro-Glu-Glu-Tyr-Leu-OH**

Analyse :

| | Asp | Glu | Gly | 4-Abu | Phe | Leu + Ile + Abo | Tyr |
|---|---|---|---|---|---|---|---|
| calculé | 1 | 3 | 1 | 1 | 1 | 3 | 1 |
| trouvé | 1,00 | 3,06 | 0,95 | 0,99 | 1,08 | 2,88 | 1,02 |

Spectre de masse (FAB) : $MH^+$, m/z = 1433

**EXEMPLE 23 : H-Arg-Gly-Asp-Phe-Glu-Abo-Ile-Pro-Glu-Glu-Tyr$\Psi$($CH_2$NH)Leu-OH**

Analyse :

| | Arg | Asp | Glu | Gly | Pro | Phe | Ile + Abo |
|---|---|---|---|---|---|---|---|
| calculé | 1 | 1 | 3 | 1 | 1 | 1 | 2 |
| trouvé | 1,07 | 1,06 | 2,97 | 0,92 | 1,01 | 0,99 | 2,01 |

Spectre de masse (FAB) : $MH^+$, m/z = 1489

**EXEMPLE 24 : $X_1$-Gly-Asp-Phe-Glu-Abo-Ile-Pro-Glu-Glu-Tyr-Leu-OH**

Analyse :

| | Asp | Glu | Gly | Pro | Phe | Ile + Abo | Leu | Tyr |
|---|---|---|---|---|---|---|---|---|
| calculé | 1 | 3 | 1 | 1 | 1 | 2 | 1 | 1 |
| trouvé | 1,04 | 3,02 | 1,06 | 1,05 | 1,01 | 1,98 | 1,07 | 0,95 |

Spectre de masse (FAB) : $MH^+$, m/z = 1508

EXEMPLE 25 : **$CH_3CO$-Arg-Gly-Asp-Phe-Glu-Abo-Ile-Pro-Glu-Glu-Tyr-Leu-OH**

Analyse :

| | Arg | Asp | Glu | Gly | Pro | Phe | Ile + Abo | Leu | Tyr |
|---|---|---|---|---|---|---|---|---|---|
| calculé | 1 | 1 | 3 | 1 | 1 | 1 | 2 | 1 | 1 |
| trouvé | 1,00 | 1,00 | 3,02 | 1,04 | 1,08 | 0,98 | 1,82 | 1,10 | 1,01 |

Spectre de masse (FAB) : $MH^+$, m/z = 1546

**EXEMPLE 26 : Arg-Gly-Asp-Phe-Glu-Abo-Ile-Pro-Glu-Glu-Tyr-Leu-OH**

Analyse :

| | Arg | Asp | Glu | Gly | Pro | Phe | Ile + Abo | Leu | Tyr |
|---|---|---|---|---|---|---|---|---|---|
| calculé | 1 | 1 | 3 | 1 | 1 | 1 | 2 | 1 | 1 |
| trouvé | 1,04 | 1,02 | 3,03 | 0,99 | 1,07 | 1,03 | 1,94 | 1,02 | 0,97 |

Spectre de masse (FAB) : $MH^+$, m/z = 1504

**EXEMPLE 27 : phe-Pro-Arg-Abu-Glu-Abo-Ile-Pro-Glu-Glu-tyr-Leu-OH**

Analyse :

| | Arg | Glu | Abu | Pro | Phe | Ile | Leu + Abo | Tyr |
|---|---|---|---|---|---|---|---|---|
| calculé | 1 | 3 | 1 | 2 | 1 | 1 | 2 | 1 |
| trouvé | 1,00 | 2,83 | 0,95 | 2,09 | 1,03 | 1,05 | 2,00 | 0,99 |

Spectre de masse (FAB) : $MH^+$, m/z = 1514

**EXEMPLE 28 : $X_1$-Gly-Asp-Phe-Glu-Abo-Ile-Pro-Glu-Glu-Tyr-Leu-glu-OH**

Analyse :

| | Asp | glu | Gly | Pro | Phe | Ile | Leu + Abo | Tyr |
|---|---|---|---|---|---|---|---|---|
| calculé | 1 | 4 | 1 | 1 | 1 | 1 | 2 | 1 |
| trouvé | 1,08 | 4,21 | 1,02 | 1,10 | 0,99 | 0,90 | 1,64 | 1,04 |

Spectre de masse (FAB) : $MH^+$, m/z = 1638

**EXEMPLE 29 : Suc-Glu-Tyr-Abo-Ile-Pro-Glu-Glu-Ala-Cha-glu-OH**

Analyse :

| | Glu | Ala | Pro | Ile | Abo | Cha | Tyr |
|---|---|---|---|---|---|---|---|
| calculé | 4 | 1 | 1 | 1 | 1 | 1 | 1 |
| trouvé | 4,15 | 0,99 | 1,00 | 0,99 | 0,95 | 0,94 | 0,97 |

Spectre de masse (FAB) : $MH^+$, m/z = 1369

**EXEMPLE 30 : Suc-Tyr-Glu-Pro-Ile-Oic-Glu-Glu-Ala-Thi-glu-OH**

Analyse :

| | Glu | Ala | Pro | Ile | Oic | Tyr | Thi |
|---|---|---|---|---|---|---|---|
| calculé | 4 | 1 | 1 | 1 | 1 | 1 | 1 |
| trouvé | 4,34 | 0,93 | 1,05 | 0,89 | 0,96 | 0,95 | 0,89 |

Spectre de masse (FAB) : $MH^+$, m/z = 1383

**EXEMPLE 31 : Suc-Tyr-Glu-Pro-Ile-Pro-Glu-Glu-Ala-Oic-glu-OH**

Analyse :

| | Glu | Ala | Pro | Ile | Oic | Tyr |
|---|---|---|---|---|---|---|
| calculé | 4 | 1 | 2 | 1 | 1 | 1 |
| trouvé | 3,91 | 0,96 | 2,04 | 1,12 | 0,99 | 0,97 |

Spectre de masse (FAB) : $MH^+$, m/z = 1327

**EXEMPLE 32 : $X_1$-Gly-Asp-Phe-Glu-Abo-Ile-Pro-Glu-Glu-Ala-Oic-glu-OH**

Analyse :

| | Asp | Glu | Gly | Ala | Pro | Phe | Ile + Abo | Oic |
|---|---|---|---|---|---|---|---|---|
| calculé | 1 | 4 | 1 | 1 | 1 | 1 | 2 | 1 |
| trouvé | 1,09 | 4,25 | 0,87 | 0,95 | 0,85 | 0,86 | 2,23 | 0,90 |

Spectre de masse (FAB) : $MH^+$, m/z = 1584

**EXEMPLE 33 : Suc-Tic-Glu-Pro-Ile-Pro-Glu-Glu-Ala-Oic-glu-OH**

Analyse :

| | Glu | Ala | Pro | Ile | Oic | Tic |
|---|---|---|---|---|---|---|
| calculé | 4 | 1 | 2 | 1 | 1 | 1 |
| trouvé | 4,08 | 1,07 | 1,92 | 0,92 | 0,91 | 1,09 |

Spectre de masse (FAB) : $MH^+$, m/z = 1323

**EXEMPLE 34 : Suc-tic-Glu-Pro-Ile-Pro-Glu-Glu-Ala-Oic-glu-OH**

Analyse :

| | Glu | Ala | Pro | Ile | Oic | tic |
|---|---|---|---|---|---|---|
| calculé | 4 | 1 | 2 | 1 | 1 | 1 |
| trouvé | 3,86 | 1,11 | 2,00 | 0,96 | 0,95 | 1,07 |

Spectre de masse (FAB) : $MH^+$, m/z = 1323

**EXEMPLE 35 : nal-Asp-Phe-Glu-Abo-Ile-Pro-Glu-Asp-Tyr-Leu-OH**

Analyse :

| | Asp | Glu | Pro | Phe | Ile | Leu | nal | Tyr |
|---|---|---|---|---|---|---|---|---|
| calculé | 2 | 2 | 1 | 1 | 1 | 2 | 1 | 1 |
| trouvé | 1,97 | 2,14 | 1,07 | 1,01 | 0,96 | 1,90 | 1,07 | 0,90 |

Spectre de masse (FAB) : $MH^+$, m/z = 1474

**EXEMPLE 36 : Z-Asp-Phe-Glu-Abo-Ile-Pro-Glu-Asp-Tyr-nal-nal-Leu-OH**

Analyse :

| | Asp | Glu | Pro | Phe | Ile + Abo | Leu | nal | Tyr |
|---|---|---|---|---|---|---|---|---|
| calculé | 2 | 2 | 1 | 1 | 2 | 1 | 2 | 1 |
| trouvé | 2,06 | 2,20 | 1,04 | 1,02 | 1,82 | 1,10 | 1,85 | 0,91 |

Spectre de masse (FAB) : $MH^+$, m/z = 1804

**EXEMPLE 37 : Z-Asp-Phe-Glu-Abo-Ile-Abo-Glu-Asp-Tyr-leu-OH**

Analyse :

| | Asp | Glu | Phe | Ile + Abo | Leu | Tyr |
|---|---|---|---|---|---|---|
| calculé | 2 | 2 | 1 | 3 | 1 | 1 |
| trouvé | 2,10 | 2,19 | 0,96 | 2,85 | 1,09 | 1,10 |

Spectre de masse (FAB) : $MH^+$, m/z = 1450

**EXEMPLE 38 : H-Asp-Phe-Glu-Orn-Ile-Pro-Asp-Glu-Tyr(Bzl)-Leu-$NH_2$**

Analyse :

| | Asp | Glu | Pro | Phe | Ile | Leu | Orn | Tyr |
|---|---|---|---|---|---|---|---|---|
| calculé | 2 | 2 | 1 | 1 | 1 | 1 | 1 | 1 |
| trouvé | 1,80 | 1,84 | 0,92 | 0,90 | 0,87 | 0,92 | 1,08 | 1,03 |

Spectre de masse (FAB) : $MH^+$, m/z = 1324

**EXEMPLE 39 : $X'_0$-Gly-Asp-Phe-Glu-Abo-Ile-Pro-Glu-Glu-Tyr-Leu-OH**

Analyse :

| | Asp | Glu | Gly | Pro | Phe | Ile | Leu + Abo | Tyr |
|---|---|---|---|---|---|---|---|---|
| calculé | 1 | 3 | 1 | 1 | 1 | 1,0 | 2 | 1 |
| trouvé | 1,5 | 3,19 | 1,06 | 0,97 | 1,00 | 0,90 | 1,86 | 0,55 |

Spectre de masse (FAB) : $MH^+$, m/z = 1489

**EXEMPLE 40 : $X_1$-Gly-Asp-Phe-Glu-Abo-Ile-Pro-Glu-Glu-Pcp-Leu-OH**

**EXEMPLE 41 : $X_1$-Gly-Asp-Phe-Glu-Abo-Ile-Pro-Glu-Glu-Pcp-Leu-glu-OH**

**EXEMPLE 42 : $X_1$-Gly-Asp-Phe-Glu-Abo-Ile-Pro-Glu-Glu-Pcp-leu-OH**

**EXEMPLE 43 : $X_1$-Gly-Asp-Phe-Glu-Abo-Ile-Pro-Glu-Glu-Pcp-Leu-Pro-OH**

**EXEMPLE 44 : $X_1$-Gly-Asp-Phe-Glu-Abo-Ile-Pro-Glu-Glu-Pcp-Leu-pro-OH**

**EXEMPLE 45 : $X_1$-Gly-Asp-Phe-Glu-Abo-Ile-Pro-Glu-Glu-Pcp-Leu-ol**

**EXEMPLE 46 : $X_1$-Gly-Asp-Phe-Glu-Abo-Ile-Pro-Glu-Glu-Pcp-Leu-βAla-OH**

**EXEMPLE 47 : $X_1$-Gly-Asp-Phe-Glu-Abo-Ile-Pro-Glu-Glu-($pCH_2PO_3$)Phe-Leu-OH**

**EXEMPLE 48 : $X_1$-Gly-Asp-Phe-Glu-Abo-Ile-Pro-Glu-Glu-($pCH_2PO_3H$)Phe-Leu-glu-OH**

**EXEMPLE 49 : $X_1$-Gly-Asp-Phe-Glu-Abo-Ile-Pro-Glu-Glu-($pCH_2PO_3H$)PheLeu-βAla-OH**

**EXEMPLE 50 : $X_1$-Gly-Asp-Phe-Glu-Abo-Ile-Pro-Glu-Glu-($pCH_2PO_3H$)Phe-Leu-4Abu-OH**

**EXEMPLE 51 : $X_7$-Phe-Glu-Abo-Ile-Pro-Glu-Glu-($pCH_2PO_3H$)Phe-Leu-glu-OH**

**EXEMPLE 52 : $X_7$-Glu-Abo-Ile-Pro-Glu-Glu-($pCH_2PO_3H$)Phe-Leu-glu-OH**

**EXEMPLE 53 : $X_7$-Phe-Glu-Abo-Ile-Pro-Glu-Glu-($pCH_2PO_3H$)Phe-Leu-βAla-OH**

**EXEMPLE 54 : $X_8$-Phe-Glu-Abo-Ile-Pro-Glu-Glu-($pCH_2PO_3H$)Phe-Leu-glu-OH**

ETUDE PHARMACOLOGIQUE DES COMPOSES DE L'INVENTION

**EXEMPLE 55 : Activité antithrombotique**

Le modèle de thrombose expérimentale utilisé est celui d'une thrombose veineuse induite chez le rat Wistar mâle par ligature de la veine cave inférieure sous la veine rénale gauche.

Les animaux à tester sont anesthésiés par administration intrapéritonéale de Brietal® (méthohexital sodique) à la dose de 1 ml/kg.

La ligature est alors réalisée. Le produit à tester est injecté 2 heures plus tard par voie sous cutanée dans la région sous clavière gauche. 4 heures après cette injection, le sang est prélevé par voie intracardiaque directe, la veine est excisée et le caillot est récupéré puis pesé après passage dans une étuve à 37°C pendant 12 heures.

Les composés de l'invention ont été testés à une dose unique de 250 μg/kg par rapport à de l'hirudine non sulfatée utilisée comme composé témoin.

Les résultats de ce test ont montré que les composés de l'invention permettent une diminution du poids du caillot 3 à 4 fois plus importante que celle observée lors de l'injection d'hirudine non sulfatée.

**EXEMPLE 56 : Activité anticoagulante, mesure du temps de thrombine**

En présence d'une quantité standard de thrombine, un plasma normal coagule en un temps défini et constant, appelé temps de thrombine (TT). Tout allongement de ce temps traduit une anomalie de la fibrinoformation (coagulation).

Les rats Sprague-Dawley sont anesthésiés avec du pentobarbital sodique (60 mg/kg, i.p.), l'artère carotide est cathétérisée et les prélèvements sanguins réalisés dans une solution de citrate trisodique (0.109 M). Un plasma pauvre en plaquettes est obtenu par centrifugation des échantillons sanguins (3000 g, 15 min.). Le plasma peut être conservé 8 heures à 20°C.

Le temps de thrombine est réalisé avec le réactif thrombine Prest et déterminé automatiquement en utilisant un coagulomètre.

L'antagoniste ou le solvant (10 μl) est additionné au plasma (90 μl), puis incubé 2 minutes à 37°C. 100 μl de thrombine sont ajoutés en déclenchant le chronomètre.

Dans nos conditions, les TT obtenus dans le plasma témoin sont de l'ordre de 30 secondes. L'activité d'un antagoniste est évaluée par sa capacité à prolonger ce temps de thrombine par rapport au témoin.

Dans ces conditions, les composés de l'invention permettent une prolongation du temps de thrombine de 3 à 5 fois. Cette prolongation est 2 à 3 fois supérieure à celle obtenue avec l'hirudine 55-65 non sulfatée.

C'est le cas plus particulièrement des composés des exemples 24, 28 et 30.

**EXEMPLE 57 : Agrégation plaquettaire**

Dans ce test, l'activité antithrombine des antagonistes est évaluée en mesurant l'inhibition de l'agrégation plaquettaire induite par la thrombine.

Les lapins (2-3 kg) sont anesthésiés avec du sodium pentobarbital (30 mg/kg, i.v.). Après cannulation de l'artère carotide gauche, le sang est prélevé sur citrate de sodium (0.109 M) (1 vol de citrate pour 9 vol de sang).

Le plasma riche en plaquettes (PRP) est obtenu par centrifugation (20°C) à 250 g pendant 20 minutes et le plasma pauvre en plaquettes (PPP) par centrifugation à 1000 g pendant 10 minutes. Le nombre des plaquettes est ajusté entre 300-350 000 pl/mm3 par dilution en PPP autologue. Le PRP est conservé à la température de la pièce jusqu'au moment du test et est utilisé dans les 4 heures qui suivent le prélèvement.

Le PRP est de nouveau centrifugé (900 g, 15 minutes) et les plaquettes sont lavées dans une solution saline tamponnée avec du Tris, contenant de la gélatine (0.2 %). Les plaquettes lavées (PL) sont ensuite resuspendues dans une solution physiologique et conservées à la température de la pièce.

L'agrégation plaquettaire est réalisée à 37°C dans des tubes en verre siliconés à l'aide d'un agrégomètre. Le PRP et les PL sont agités à 1000 rpm (révolutions par minute).

La thrombine est incubée 3 minutes à 37°C avec la substance à tester (à différentes concentrations) ou avec le solvant dans un volume de 50 μl. Les PL (225 μl) sont préincubées 1 minute à 37°C dans une cuvette en verre siliconée sous agitation (1000 rpm). L'agrégation est obtenue par addition de 25 μl de la solution contenant la thrombine et la substance à tester. La réponse agrégante est enregistrée pendant au moins 5 minutes.

L'intensité de l'agrégation plaquettaire est établie en prenant l'amplitude maximale des tracés agrégants et est exprimée en pourcentage de transmission lumineuse (%T). Pour chacun des composés testés, une $IC_{50}$ a été déterminée.

Les composés de l'invention inhibent de façon dose dépendante l'agrégation plaquettaire induite par la thrombine. Les $IC_{50}$ obtenues sont de 4 à 20 fois inférieures à la valeur obtenue avec l'hirudine non sulfatée. C'est le cas plus particulièrement des composés des exemples 22, 24, 26 et 39.

**EXEMPLE 58 : Mesure de l'activité anticoagulante ex vivo. Temps de prothrombine**

Les rats OFA, à jeun ou pas, sont anesthésiés au pentobarbital (60 mg/kg, i.p.). La carotide et la jugulaire sont dégagées et catétherisées. Les catéthers sont purgés avec du serum physiologique citraté (1/40). Après installation des catéthers, un prélèvement de 1,5 $cm^3$ de sang artériel est effectué sur citrate 0.109 M (1/9).

30 minutes après, le produit à tester est administré sous un volume de 1 ml en i.v.

Des prélèvements artériels (1,5 ml) sont alors effectués à 1 min 30, 3 min, 5 min, 15 min, 30 min et 60 min.

A chaque prélèvement 1,5 ml de serum physiologique citraté est réinjecté à l'animal via la carotide.

Les tubes de sang sont centrifugés 15 min à 4000 rpm (préparation de plasma).

100 μl de plasma sont incubés avec 200 μl de néoplastine calcique. Le temps d'apparition du phénomène de coagulation est mesuré.

Les composés de l'invention, testés à des doses de 4 à 16 mg/kg, augmentent de façon dose dépendante le temps de prothrombine (TP). Leur effet est plus important que celui de l'hirudine non sulfatée. En effet, ils augmentent le TP de 1,4 à 1,8 fois. D'autre part, alors que l'activité in vivo de l'hirudine est de courte durée (30 minutes), l'augmentation du TP notée avec les composés de l'invention est toujours significative 60 minutes après leur administration.

C'est le cas, plus particulièrement, des composés des exemples 22, 24, 28, 30 et 39.

## Revendications

**1/** Composé de formule générale (I) :

$$X\text{-}A_1\text{-}A_2\text{-}A_3\text{-}A_4\text{-}A_5\text{-}A_6\text{-}A_7\text{-}A_6\text{-}A_9\text{-}A_{10}\text{-}Y \qquad (I)$$

dans laquelle :

X représente -

un groupement amino terminal éventuellement substitué par 1 ou 2 groupements alkyls ($C_1$-$C_6$) linéaires ou ramifiés, un groupement acyl ($C_1$-$C_6$) linéaire ou ramifié, un groupement benzyloxycarbonyl (Z), un groupement tert-butoxycarbonyl (Boc), un groupement 9-flurénylméthyloxycarbonyl (Fmoc), un groupement guanidinoacyl ($C_1$-$C_6$) linéaire ou ramifié tel que les groupements

$X_0$ = 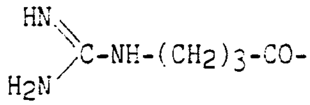 ,

ou $X'_0$ = 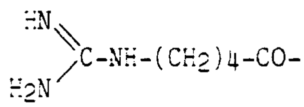 ,

un groupement guanidinoalkyl ($C_1$-$C_6$) linéaire ou ramifié, un groupement pyroglutamyl (Clp), un groupement succinyle (Suc), ou l'un quelconque des groupements suivants :

$X_1$ = 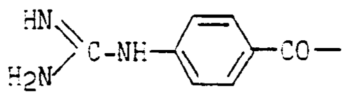

$X_2$ = 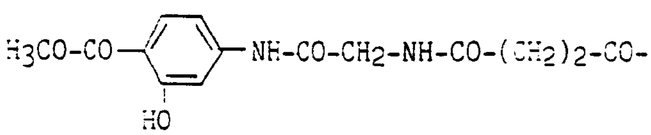

$X_3$ = 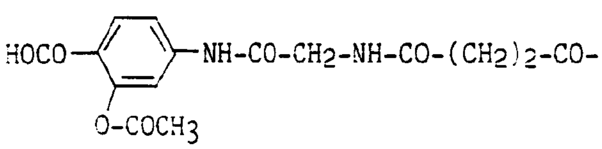

X4 = 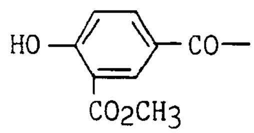

X5 = 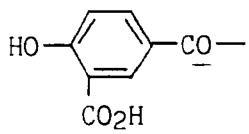

X6 = 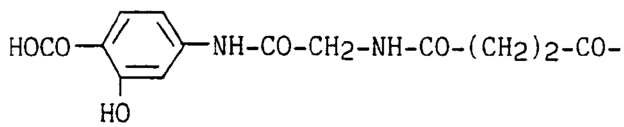

X7 = 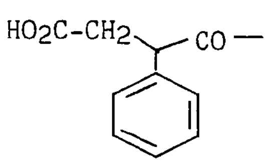

X8 = 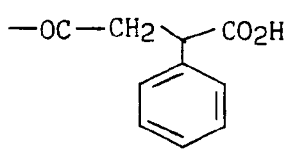

$A_1$ représente une liaison ou un résidu peptidique contenant de 1 à 3 amino-acides quelconques,

$A_2$ représente une liaison ou un résidu phénylalanine (Phe), tyrosine (Tyr), isoleucine (Ile), norvaline (Nva), (1,2,3,4)-tétrahydroisoquinoline-3-carbonyl (Tic), un résidu acide α-aminobutyrique (Abu), acide glutamique (Glu), ou un résidu d'acide aminé contenant un groupement aryle,

A3 représente une liaison ou un résidu acide glutamique (Glu), acide aspartique (Asp), β-alanine (βAla) ou tyrosine (Tyr),

$A_4$ représente une liaison ou un résidu acide glutamique (Glu), acide aspartique (Asp), proline (Pro), acide 2,3-diaminopropionique (Dpr), acide 2,4-diaminobutyrique (Dab), ornithine (Orn), lysine (Lys), 2-azabicyclo [2.2.2] octane-3-carbonyl (Abo), 2-azabicyclo [2.2.1] heptane-3-carbonyl (Abh),

$A_5$ représente une liaison ou un résidu isoleucine (Ile), norvaline (Nva), phénylalanine (Phe), proline (Pro), ornithine (Orn) ou acide 2,3-diaminopropionique (Dpr),

$A_5$ représente un résidu proline (Pro), isoleucine (Ile), norvaline (Nva), phénylalanine (Phe), ornithine (Orn), 2-azabicyclo [2.2.2] octane-3-carbonyl (Abo), 2-azabicyclo [2.2.1] heptane-3-carbonyl (Abh), octahydroindole-2-carbonyl (Oic) ou 3,4-déhydroproline (dhPro),

$A_7$ représente un résidu acide glutamique (Glu) ou acide aspartique (Asp),

$A_5$ représente un résidu acide glutamique (Glu), acide aspartique (Asp), acide 2,3-diaminopropionique (Dpr), acide 2,4-diaminobutyrique (Dab) ou β-alanine (βAla),

$A_9$ représente un résidu tyrosine (Tyr) éventuellement protégé par un groupement benzyle (Tyr(Bzl)) ou acétyle (Tyr($COCH_3$), phénylalanine (Phe), alanine (Ala), isoleucine (Ile), norvaline (Nva), p-carboxyphénylalanine (Pcp), 3-carboxytyrosine ou 4-0-acétyl-3-carboxytyrosine, (p-$CH_2PO_3H$) phénylalanine ((p$CH_2PO_3H$)Phe),

$A_{10}$ représente une liaison, un résidu leucine (Leu), valine (Val),β-naphtylalanine (Nal), cyclohexylalanine (Cha), β-(2-thiényl)alanine (Thi), octahydroindole-2-carbonyl (Oic), un résidu dipeptidique tel que Leu-Glu, Leu-Pro, Leu-βAla, Val-Glu, Nal-Glu, Cha-Glu- Thi-Glu, Oic-Glu, leucine-acide 4-aminobutyrique (Leu-4Abu), ou un résidu tripeptidique tel que Nal-Nal-Leu,

Y représente
– un groupement carboxy terminal éventuellement substitué par un groupement alkoxy ($C_1$-$C_6$) linéaire ou ramifié ou un groupement amino lui-même éventuellement substitué par un ou deux groupements alkyles ($C_1$-$C_6$) linéaires ou ramifiés,
ou bien
– un groupement carboxy terminal réduit en alcool correspondant (ol),
et comprenant au moins :
– un résidu d'amino-acide non naturel tel que le 2-azabicyclo [2.2.2] octane-3-carbonyl (Abo), le 2-azabicyclo [2.2.1] heptane-3-carbonyl (Abh), l'octahydroindole-2-carbonyl (Oic),
et/ou
– une liaison pseudopeptidique telle que $-CH_2$-NH-, $-CH_2$-S-, $-CH_2$-SO-, $-CH_2$-$SO_2$-, -NH-CO-, -CH=CH- remplaçant une liaison peptidique -CO-NH-,
et/ou
– une cyclisation non cystéinique entre les chaînes latérales de deux amino-acides, ou entre un groupement amino terminal et une chaîne latérale d'un amino-acide ou entre un groupement carboxy terminal et une chaîne latérale d'un amino-acide ou entre un groupement amino terminal et un groupement carboxy terminal,
leurs sels d'addition à un acide ou à une base pharmaceutiquement acceptable,
chaque amino-acide de la séquence peptidique étant optiquement pur et le carbone $\alpha$ de chaque amino-acide ayant la configuration D ou L.

**2/** Composés selon la revendication 1 tels qu'ils comprennent au moins un résidu d'amino-acide non naturel tel que le 2-azabicyclo [2.2.2] octane-3-carbonyl (Abo), le 2-azabicyclo [2.2.1] heptane-3-carbonyl (Abh) ou l'octahydroindole-2-carbonyl (Oic).

**3/** Composés selon la revendication 1 tels qu'ils comprennent au moins une liaison pseudopeptidique telle que $-CH_2$-NH-, $-CH_2$-S-, $-CH_2$-SO-, $-CH_2$-$SO_2$-, -NH-CO-, -CH=CH- remplaçant une liaison peptidique -CO-NH-

**4/** Composés selon la revendication 1 tels qu'ils comprennent au moins une cyclisation non cystéinique entre les chaînes latérales de deux amino-acides, ou entre un groupement amino terminal et une chaîne latérale d'un amino-acide ou entre un groupement carboxy terminal et une chaîne latérale d'un amino-acide ou entre un groupement amino terminal et un groupement carboxy terminal.

**5/** Composé selon l'une quelconque des revendications 1 ou 2 qui est le $X_1$-Gly-Asp-Phe-Glu-Abo-Ile-Pro-Glu-Glu-Tyr-Leu-OH, $X_1$ représentant un radical

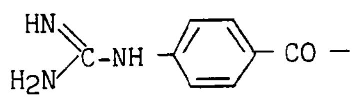 ,

Abo un résidu 2-azabicyclo[2.2.2]octane-3-carbonyl.

**6/** Composé selon l'une quelconque des revendications 1 ou 2 qui est le $X_1$-Gly-Asp-Phe-Glu-Abo-Ile-Pro-Glu-Glu-Tyr-Leu-glu-OH, $X_1$ représentant un radical

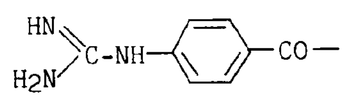 ,

Abo un résidu 2-azabicyclo[2.2.2]octane-3-carbonyl.

**7/** Composé selon l'une quelconque des revendications 1 ou 2 qui est le suc-Tyr-Glu-Pro-Ile-Oic-Glu-Glu-Ala-Thi-glu-OH, Oic représentant un résidu octahyriroindole-2-carbonyl, Thi un résidu $\beta$-(2-thiényl)alanine.

**8/** Composé selon l'une quelconque des revendications 1 ou 2 qui est le H-4Abu-Gly-Asp-Phe-Glu-Abo-Ile-Pro-Glu-Glu-Tyr-Leu-OH, 4Abu représentant un résidu d'acide 4-aminobutyrique et Abo un résidu 2-azabicyclo[2.2.2]octane-3-carbonyl.

**9/** Composé selon l'une quelconque des revendications 1 ou 2 qui est le $X'_0$-Gly-Asp-Phe-Glu-Abo-Ile-Pro-Glu-Glu-Tyr-Leu-OH, $X'_0$ représentant un radical

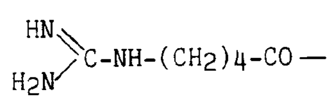

et Abo un résidu 2-azabicyclo[2.2.2]octane-3-carbonyl.

**10/** Procédé de préparation des dérivés de formule (I) caractérisé en ce qu'ils peuvent être obtenus par synthèse séquentielle sur phase solide d'amino-acides protégés, par synthèse de fragments peptidiques en solution,
éventuellement par combinaison de ces deux techniques,
si on le désire par cyclisation totale ou partielle à travers deux fonctions déprotégées de façon sélective et capable de former une liaison covalente,
et si on le souhaite par introduction,
selon les techniques classiques de la chimie organique, d'une liaison pseudopeptidique à tout moment de synthèse de la séquence peptidique,
dérivés de formule (I) que l'on transforme, si nécessaire, en leurs sels d'addition à un acide ou à une base pharmaceutiquement acceptable.

**11/** Compositions pharmaceutiques contenant comme principe actif au moins un composé selon l'une quelconque des revendications 1 à 9, seul ou en combinaison avec un ou plusieurs excipients ou véhicules inertes, non toxiques, pharmaceutiquement acceptables.

**12/** Compositions pharmaceutiques selon la revendication 11 contenant au moins un principe actif selon l'une quelconque des revendications 1 à 9 utiles comme anticoagulants, dans le traitement des thromboses veineuses aigües, de l'embolie pulmonaire, dans l'embolie artérielle des extrêmités, de l'infarctus du myocarde, de l'athérosclérose et des manifestations thromboemboliques, ainsi que pour maintenir l'homéostasie sanguine en particulier dans la circulation extracorporelle.

**Revendications pour l'Etat contractant suivant : ES**

**1/** Procédé de préparation des composés de formule générale (I) :

$$X\text{-}A_1\text{-}A_2\text{-}A_3\text{-}A_4\text{-}A_5\text{-}A_6\text{-}A_7\text{-}A_6\text{-}A_9\text{-}A_{10}\text{-}Y \qquad (I)$$

dans laquelle :

X représente -

un groupement amino terminal éventuellement substitué par 1 ou 2 groupements alkyls ($C_1$-$C_6$) linéaires ou ramifiés, un groupement acyl ($C_1$-$C_6$) linéaire ou ramifié, un groupement benzyloxycarbonyl (Z), un groupement tert-butoxycarbonyl (Boc), un groupement 9-fluorénylméthyloxycarbonyl (Fmoc), un groupement guanidinoacyl ($C_1$-$C_6$) linéaire ou ramifié tel que les groupements

$X_0$ = 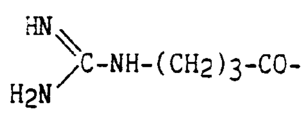 ,

ou $X'_0$ = 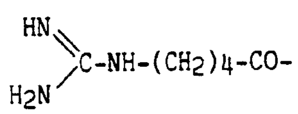 ,

un groupement guanidinoalkyl ($C_1$-$C_6$) linéaire ou ramifié, un groupement pyroglutamyl (Glp), un groupement succinyle (Suc), ou l'un quelconque des groupements suivants :

$X_1$ = 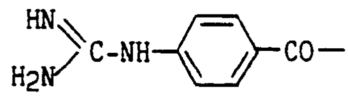

$X_2$ = 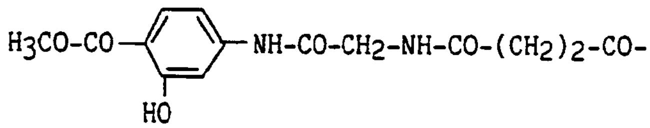

$X_3$ = 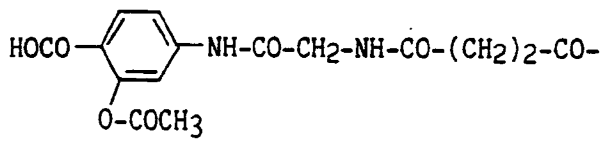

$X_4$ = 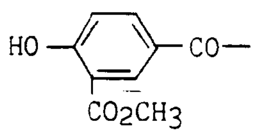

$X_5$ = 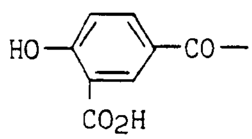

$X_6$ = 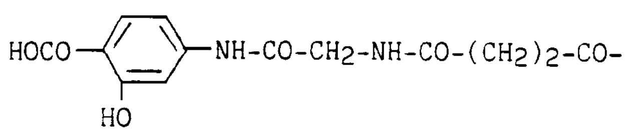

$X_7$ = 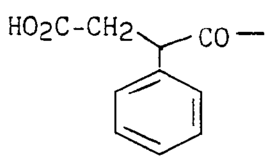

$X_8$ = 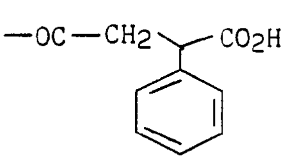

$A_1$ représente une liaison ou un résidu peptidique contenant de 1 à 3 amino-acides quelconques,

$A_2$ représente une liaison ou un résidu phénylalanine (Phe), tyrosine (Tyr), isoleucine (Ile), norvaline (Nva), (1,2,3,4)-tétrahydroisoquinoline-3-carbonyl (Tic), un résidu acide α-aminobutyrique (Abu), acide glutamique (Glu), ou un résidu d'acide aminé contenant un groupement aryle,

$A_3$ représente une liaison ou un résidu acide glutamique (Glu), acide aspartique (Asp), β-alanine (βAla) ou tyrosine (Tyr),

$A_4$ représente une liaison ou un résidu acide glutamique (Glu), acide aspartique (Asp), proline (Pro),

acide 2,3-diaminopropionique (Dpr), acide 2,4-diaminobutyrique (Dab), ornithine (Orn), lysine (Lys), 2-azabicyclo [2.2.2] octane-3-carbonyl (Abo), 2-azabicyclo [2.2.1] heptane-3-carbonyl (Abh),

$A_5$ représente une liaison ou un résidu isoleucine (Ile), norvaline (Nva), phénylalanine (Phe), proline (Pro), ornithine (Orn) ou acide 2,3-diaminopropionique (Dpr),

$A_5$ représente un résidu proline (Pro), isoleucine (Ile), norvaline (Nva), phénylalanine (Phe), ornithine (Orn), 2-azabicyclo [2.2.2] octane-3-carbonyl (Abo), 2-azabicyclo [2.2.1] heptane-3-carbonyl (Abh), octahydroindole-2-carbonyl (Oic) ou 3,4-déhydroproline (dhPro),

$A_7$ représente un résidu acide glutamique (Glu) ou acide aspartique (Asp),

$A_5$ représente un résidu acide glutamique (Glu), acide aspartique (Asp), acide 2,3-diaminopropionique (Dpr), acide 2,4-diaminobutyrique (Dab) ou β-alanine (βAla),

$A_9$ représente un résidu tyrosine (Tyr) éventuellement protégé par un groupement benzyle (Tyr(Bzl)) ou acétyle (Tyr($COCH_3$), phénylalanine (Phe), alanine (Ala), isoleucine (Ile), norvaline (Nva), p-carboxyphénylalanine (Pcp), 3-carboxytyrosine ou 4-0-acétyl-3-carboxytyrosine, (p-$CH_2PO_3H$) phénylalanine ((p$CH_2PO_3H$)Phe),

$A_{10}$ représente une liaison, un résidu leucine (Leu), valine (Val), β-naphtylalanine (Nal), cyclohexylalanine (Cha), β-(2-thiényl)alanine (Thi), octahydroindole-2-carbonyl (Oic), un résidu dipeptidique tel que Leu-Glu, Leu-Pro, Leu-βAla, Val-Glu, Nal-Glu, Cha-Glu- Thi-Glu, Oic-Glu, leucine-acide 4-aminobutyrique (Leu-4Abu), ou un résidu tripeptidique tel que Nal-Nal-Leu,

Y représente

– un groupement carboxy terminal éventuellement substitué par un groupement alkoxy ($C_1$-$C_6$) linéaire ou ramifié ou un groupement amino lui-même éventuellement substitué par un ou deux groupements alkyles ($C_1$-$C_6$) linéaires ou ramifiés,

ou bien

– un groupement carboxy terminal réduit en alcool correspondand (ol),

et comprenant au moins :

– un résidu d'amino-acide non naturel tel que le 2-azabicyclo [2.2.2] octane-3-carbonyl (Abo), le 2-azabicyclo [2.2.1] heptane-3-carbonyl (Abh), l'octahydroindole-2-carbonyl (Oic),

et/ou

– une liaison pseudopeptidique telle que -$CH_2$-NH-, -$CH_2$-S-, -$CH_2$-SO-, -$CH_2$-$SO_2$-, -NH-CO-, -CH=CH- remplaçant une liaison peptidique -CO-NH-,

et/ou

– une cyclisation non cystéinique entre les chaînes latérales de deux amino-acides, ou entre un groupement amino terminal et une chaîne latérale d'un amino-acide ou entre un groupement carboxy terminal et une chaîne latérale d'un amino-acide ou entre un groupement amino terminal et un groupement carboxy terminal,

leurs sels d'addition à un acide ou à une base pharmaceutiquement acceptable,

chaque amino-acide de la séquence peptidique étant optiquement pur et le carbone α de chaque amino-acide ayant la configuration D ou L,

caractérisé en ce qu'ils peuvent être obtenus par synthèse séquentielle sur phase solide d'amino-acides protégés, par synthèse de fragments peptidiques en solution,

éventuellement par combinaison de ces deux techniques, si on le désire par cyclisation totale ou partielle à travers deux fonctions déprotégées de façon sélective et capable de former une liaison covalente,

et si on le souhaite par introduction,

selon les techniques classiques de la chimie organique, d'une liaison pseudopeptidique à tout moment de synthèse de la séquence peptidique,

dérivés de formule (I) que l'on transforme, si nécessaire, en leurs sels d'addition à un acide ou à une base pharmaceutiquement acceptable.

**2/** Procédé de préparation des composés selon la revendication 1 tels qu'ils comprennent au moins un résidu d'amino-acide non naturel tel que le 2-azabicyclo [2.2.2] octane-3-carbonyl (Abo), le 2-azabicyclo [2.2.1] heptane-3-carbonyl (Abh) ou l'octahydroindole-2-carbonyl (Oic).

**3/** Procédé de préparation des composés selon la revendication 1 tels qu'ils comprennent au moins une liaison pseudopeptidique telle que -$CH_2$-NH-, -$CH_2$-S-, -$CH_2$-SO-, -$CH_2$-$SO_2$-, -NH-CO-, -CH=CH- remplaçant une liaison peptidique -CO-NH-.

**4/** Procédé de préparation des composés selon la revendication 1 tels qu'ils comprennent au moins une cyclisation non cystéinique entre les chaînes latérales de deux amino-acides, ou entre un groupement amino terminal et une chaîne latérale d'un amino-acide ou entre un groupement carboxy terminal et une chaîne latérale d'un amino-acide ou entre un groupement amino terminal et un groupement carboxy terminal.

**5/** Procédé de préparation du composé selon l'une quelconque des revendications 1 ou 2 qui est le $X_1$-

Gly-Asp-Phe-Glu-Abo-Ile-Pro-Glu-Glu-Tyr-Leu-OH, $X_1$ représentant un radical

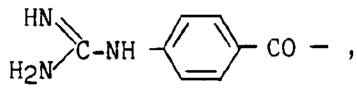

Abo un résidu 2-azabicyclo[2.2.2]octane-3-carbonyl.

**6/** Procédé de préparation du composé selon l'une quelconque des revendications 1 ou 2 qui est le $X_1$-Gly-Asp-Phe-Glu-Abo-Ile-Pro-Glu-Glu-Tyr-Leu-glu-OH, $X_1$ représentant un radical

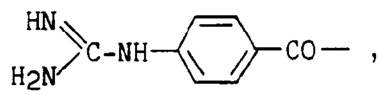

Abo un résidu 2-azabicyclo[2.2.2]octane-3-carbonyl.

**7/** Procédé de préparation du composé selon l'une quelconque des revendications 1 ou 2 qui est le suc-Tyr-Glu-Pro-Ile-Oic-Glu-Glu-Ala-Thi-glu-OH, Oic représentant un résidu octahydroindole-2-carbonyl, Thi un résidu β-(2-thiényl)alanine.

**8/** Procédé de préparation du composé selon l'une quelconque des revendications 1 ou 2 qui est le H-4Abu-Gly-Asp-Phe-Glu-Abo-Ile-Pro-Glu-Glu-Tyr-Leu-OH, 4Abu représentant un résidu d'acide 4-aminobutyrique et Abo un résidu 2-azabicyclo[2.2.2]octane-3-carbonyl.

**9/** Procédé de préparation du composé selon l'une quelconque des revendications 1 ou 2 qui est le $X'_0$-Gly-Asp-Phe-Glu-Abo-Ile-Pro-Glu-Glu-Tyn-Leu-OH, .$X'_0$ représentant un radical

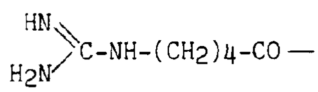

et Abo un résidu 2-azabicyclo[2.2.2]octane-3-carbonyl.

**Revendications pour l'Etat contractant suivant : GR**

**1/** Procédé de préparation des composés de formule générale (I) :

$$X\text{-}A_1\text{-}A_2\text{-}A_3\text{-}A_4\text{-}A_5\text{-}A_6\text{-}A_7\text{-}A_6\text{-}A_9\text{-}A_{10}\text{-}Y \qquad (I)$$

dans laquelle :

X représente - un groupement amino terminal éventuellement substitué par 1 ou 2 groupements alkyls ($C_1$-$C_6$) linéaires ou ramifiés, un groupement acyl ($C_1$-$C_6$) linéaire ou ramifié, un groupement benzyloxycarbonyl (Z), un groupement tert-butoxycarbonyl (Boc), un groupement 9-fluorénylméthyloxycarbonyl (Fmoc), un groupement guanidinocyl ($C_1$-$C_6$) linéaire ou ramifié tel que les groupements

$X_0$ = 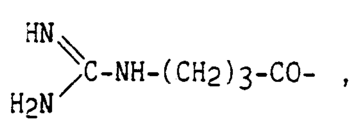 ,

ou $X'_0$ = 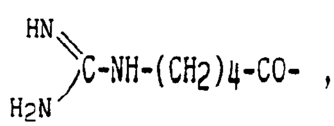 ,

un groupement guanidinoalkyl ($C_1$-$C_6$) linéaire ou ramifié, un groupement pyroglutamyl (Glp), un groupement succinyle (Suc), ou l'un quelconque des groupements suivants :

$X_1$ = 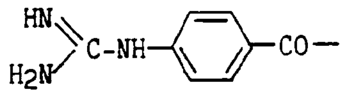

$X_2$ = 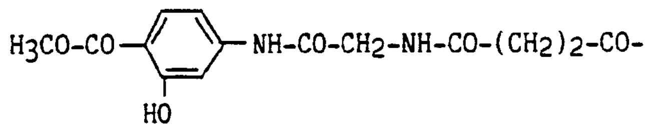

$X_3$ = 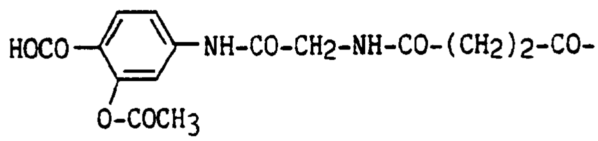

$X_4$ = 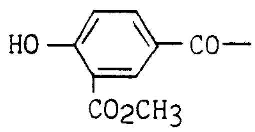

$X_5$ = 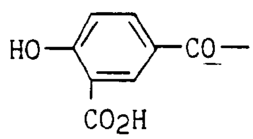

$X_6$ = 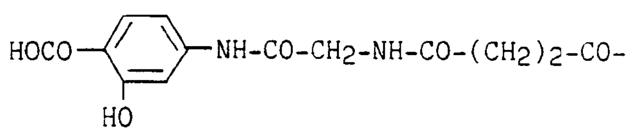

$X_7$ = 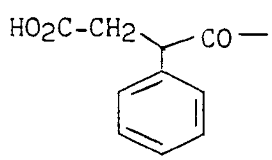

$X_8$ = 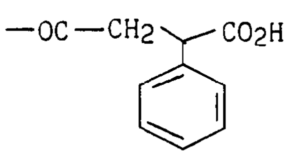

$A_1$ représente une liaison ou un résidu peptidique contenant de 1 à 3 amino-acides quelconques,

$A_2$ représente une liaison ou un résidu phénylalanine (Phe), tyrosine (Tyr), isoleucine (Ile), norvaline (Nva), (1,2,3,4)-tétrahydroisoquinoline-3-carbonyl (Tic), un résidu acide α-aminobutyrique (Abu), acide glutamique (Glu), ou un résidu d'acide aminé contenant un groupement aryle,

$A_3$ représente une liaison ou un résidu acide glutamique (Glu), acide aspartique (Asp), β-alanine (βAla) ou tyrosine (Tyr),

$A_4$ représente une liaison ou un résidu acide glutamique (Glu), acide aspartique (Asp), proline (Pro),

acide 2,3-diaminopropionique (Dpr), acide 2,4-diaminobutyrique (Dab), ornithine (Orn), lysine (Lys), 2-azabicyclo [2.2.2] octane-3-carbonyl (Abo), 2-azabicyclo [2.2.1] heptane-3-carbonyl (Abh),

$A_5$ représente une liaison ou un résidu isoleucine (Ile), norvaline (Nva), phénylalanine (Phe), proline (Pro), ornithine (Orn) ou acide 2,3-diaminopropionique (Dpr),

$A_5$ représente un résidu proline (Pro), isoleucine (Ile), norvaline (Nva), phénylalanine (Phe), ornithine (Orn), 2-azabicyclo [2.2.2] octane-3-carbonyl (Abo), 2-azabicyclo [2.2.1] heptane-3-carbonyl (Abh), octahydroindole-2-carbonyl (Oic) ou 3,4-déhydroproline (dhPro),

$A_7$ représente un résidu acide glutamique (Glu) ou acide aspartique (Asp),

$A_5$ représente un résidu acide glutamique (Glu), acide aspartique (Asp), acide 2,3-diaminopropionique (Dpr), acide 2,4-diaminobutyrique (Dab) ou β-alamine (βAla),

$A_9$ représente un résidu tyrosine (Tyr) éventuellement protégé par un groupement benzyle (Tyr(Bzl)) ou acétyle (Tyr($COCH_3$), phénylalanine (Phe), alanine (Ala), isoleucine (Ile), norvaline (Nva), p-carboxyphénylalanine (Pcp), 3-carboxytyrosine ou 4-0-acétyl-3-carboxytyrosine, (p-$CH_2PO_3H$) phénylalanine ((p$CH_2PO_3H$)Phe),

$A_{10}$ représente une liaison, un résidu leucine (Leu), valine (Val), β-naphtylalanine (Nal), cyclohexylalanine (Cha), β-(2-thiényl)alanine (Thi), octahydroindole-2-carbonyl (Oic), un résidu dipeptidique tel que Leu-Glu, Leu-Pro, Leu-βAla, Val-Glu, Nal-Glu, Cha-Glu- Thi-Glu, Oic-Glu, leucine-acide 4-aminobutyrique (Leu-4Abu), ou un résidu tripeptidique tel que Nal-Nal-Leu,

Y représente

– un groupement carboxy terminal éventuellement substitué par un groupement alkoxy ($C_1$-$C_6$) linéaire ou ramifié ou un groupement amino lui-même éventuellement substitué par un ou deux groupements alkyles ( $C_1$-$C_6$) linéaires ou ramifiés,

ou bien

– un groupement carboxy terminal réduit en alcool correspondant (ol),

<u>et comprenant au moins</u> :

– un résidu d'amino-acide non naturel tel que le 2-azabicyclo [2.2.2] octane-3-carbonyl (Abo), le 2-azabicyclo [2.2.1] heptane-3-carbonyl (Abh), l'octahydroindole-2-carbonyl (Oic),

et/ou

– une liaison pseudopeptidique telle que -$CH_2$-NH-, -$CH_2$-S-, -$CH_2$-SO-, -$CH_2$-$SO_2$-, -NH-CO-, -CH=CH- remplaçant une liaison peptidique -CO-NH-,

et/ou

– une cyclisation non cystéinique entre les chaînes latérales de deux amino-acides, ou entre un groupement amino terminal et une chaîne latérale d'un amino-acide ou entre un groupement carboxy terminal et une chaîne latérale d'un amino-acide ou entre un groupement amino terminal et un groupement carboxy terminal,

leurs sels d'addition à un acide ou à une base pharmaceutiquement acceptable,

chaque amino-acide de la séquence peptidique étant optiquement pur et le carbone α de chaque amino-acide ayant la configuration D ou L,

caractérisé en ce qu'ils peuvent être obtenus par synthèse séquentielle sur phase solide d'amino-acides protégés, par synthèse de fragments peptidiques en solution,

éventuellement par combinaison de ces deux techniques,

si on le désire par cyclisation totale ou partielle à travers deux fonctions déprotégées de façon sélective et capable de former une liaison covalente,

et si on le souhaite par introduction,

selon les techniques classiques de la chimie organique, d'une liaison pseudopeptidique à tout moment de synthèse de la séquence peptidique,

dérivés de formule (I) que l'on transforme, si nécessaire, en leurs sels d'addition à un acide ou à une base pharmaceutiquement acceptable.

**2/** Procédé de préparation des composés selon la revendication 1 tels qu'ils comprennent au moins un résidu d'amino-acide non naturel tel que le 2-azabicyclo [2.2.2] octane-3-carbonyl (Abo), le 2-azabicyclo [2.2.1] heptane-3-carbonyl (Abh) ou l'octahydroindole-2-carbonyl (Oic).

**3/** Procédé de préparation des composés selon la revendication 1 tels qu'ils comprennent au moins une liaison pseudopeptidique telle que -$CH_2$-NH-, -$CH_2$-S-, -$CH_2$-SO-, -$CH_2$-$SO_2$-, -NH-CO-, -CH=CH- remplaçant une liaison peptidique -CO-NH-.

**4/** Procédé de préparation des composés selon la revendication 1 tels qu'ils comprennent au moins une cyclisation non cystéinique entre les chaînes latérales de deux amino-acides, ou entre un groupement amino terminal et une chaîne latérale d'un amino-acide ou entre un groupement carboxy terminal et une chaîne latérale d'un amino-acide ou entre un groupement amino terminal et un groupement carboxy terminal.

**5/** Procédé de préparation du composé selon l'une quelconque des revendications 1 ou 2 qui est le $X_1$-Gly-Asp-Phe-Glu-Abo-Ile-Pro-Glu-Glu-Tyr-Leu-OH, $X_1$ représentant un radical

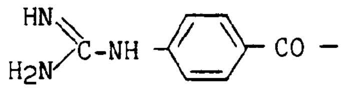 ,

Abo un résidu 2-azabicyclo[2.2.2]octane-3-carbonyl

**6/** Procédé de préparation du composé selon l'une quelconque des revendications 1 ou 2 qui est le $X_1$-Gly-Asp-Phe-Glu-Abo-Ile-Pro-Glu-Glu-Tyr-Leu-glu-OH, $X_1$ représentant un radical

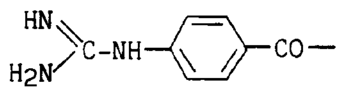 ,

Abo un résidu 2-azabicyclo[2.2.2]octane-3-carbonyl.

**7/** Procédé de préparation du composé selon l'une quelconque des revendications 1 ou 2 qui est le suc-Tyr-Glu-Pro-Ile-Oic-Glu-Glu-Ala-Thi-glu-OH, Oic représentant un résidu octahydroindole-2-carbonyl, Thi un résidu β(2-thiényl)alanine.

**8/** Procédé de préparation du composé selon l'une quelconque des revendications 1 ou 2 qui est le H-4Abu-Gly-Asp-Phe-Glu-Abo-Ile-Pro-Glu-Glu-Tyr-Leu-OH, 4Abu représentant un résidu d'acide 4-aminobutyrique et Abo un résidu 2-azabicyclo[2.2.2]octane-3-carbonyl.

**9/** Procédé de préparation du composé selon l'une quelconque des revendications 1 ou 2 qui est le $X'_0$-Gly-Asp-Phe-Glu-Abo-Ile-Pro-Glu-Glu-Tyr-Leu-OH, $X'_0$ représentant un radical

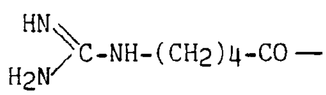

et Abo un résidu 2-azabicyclo[2.2.2]octane-3-carbonyl.